# EUROPEAN PATENT APPLICATION

(11) **EP 1 156 114 A1**
(43) Date of publication of application: **21.11.2001**
(21) Application number: 00109906.8
(22) Date of filing: 10.05.2000
(51) Int. Cl.: C12N 15/70, C12N 15/10

(54) **Vectors for use in transponson-based DNA sequencing methods**

(71) Applicant: Europäisches Laboratorium für Molekularbiologie, D-69117 Heidelberg (DE)
(72) Inventor: The designation of the inventor has not yet been filed
(74) Representative: Weiss, Wolfgang, Dipl.-Chem. Dr.

(57) **Abstract**

The present invention relates to vectors for use in transposon-based DNA sequencing methods, methods for DNA sequencing using such vectors and a method for preparing improved cloning vectors.

## Description

The present invention relates to vectors for use in transposon-based DNA sequencing methods, methods for DNA sequencing using such vectors and a method for preparing improved cloning vectors.

Nucleic acid sequencing is important both for research and diagnostic purposes. A major limitation of all transposon-based approaches to DNA sequencing is that the transposon may insert into the cloning vector rather than into the DNA to be sequenced. Although a number of methods for mapping the transposon insertion sites have been described all are very labour intensive and thus not suitable for large-scale sequencing efforts.

Transposable elements have been known for some time and been studied intensively because of their potential uses for recombinant DNA technology. In general, transposons contain inverted repeats at the termini and generate direct repeats of a short sequence at the site of insertion. All known bacterial transposons also carry enzymes which are responsible for the transposition events. These include a transposase which recognises the ends of the transposon and a resolvase which provides a site-specific recombination function.

Transposons can "jump" by two main mechanisms: conservative transposition whereby the transposing element is conserved and moves by a simple "cut-and-paste" mechanism to a target site, and replicative transposition in which the transposon is duplicated as part of its own movement yielding a co-integrate molecule in which the donor and target molecules are joined by direct repeats of the element. The resolution step involves of the breakdown of the co-integrate into a transposon-containing donor molecule and a target molecule containing a copy of the transposon. Resolution is effected by a specific transposon-encoded resolvase acting on the resolution site; it can also be accomplished by host generalised recombination. Examples of a conservatively transposing elements are Tn5, Tn7, Tn10 and Tn916, whereas *y*δ, and other members of the Tn3 family, such as Tn9/IS1 and phage Mu during lytic growth, transpose replicatively. Many transposons, e.g. the Tn transposons carry drug resistance markers.

In general, transposons are several kb (kilobases) in length and are present in the bacterial genome. The transposon *y*δ however, is present on the wild-type F factor of *E.coli* and is 6 kb in size. Transposons like *y*δ and other transposons of the Tn3 family transpose prefentially to plasmids rather than to chromosomes and show very low specificity with regard to insertion sites which is why they have been used for plasmid mutagenesis.

A known method to select for transposon insertion using *yδ* or a similar transposon is to introduce a vector into a host cell containing the wild-type F factor or pOX38::mini-*y*δ (which contains a smaller modified *y*δ transposon). This cell will support transposition and is capable of conjugative transfer of the co-integrate to an F⁻ recipient cell. A selection for the plasmid-borne marker (ampicillin resistance) can be carried out which will kill unmated recipient cells. Subsequently, resolution of the co-integrate occurs to yield a plasmid containing one copy of *y*δ or mini-*y*δ and re-forming the donor molecule (F or pOX38::mini-*y*δ), followed by growth of transconjugants. The disadvantage of this method from the view of the present invention is that the transposon may land anywhere in the vector and not just in the part which is to be sequenced.

Previously, so-called minimal vectors for negative selection of transposition events have been described (Strathmann et al., (1991) Proc. Nat. Acad. Sci. USA 88, 1247-1250; Hubbard et al., EMBL Accession Number U74374). The aim was to delete all sequences that were not absolutely necessary for cloning and into which undesirable transposition events could occur. Undesirable transposition events are such that will occur outside the inserted stretch of nucleic acid to be sequenced. In previous minimal vectors this was never achieved entirely satisfactorily since there were always sequences still present in the vector into which a transposon could insert. Strathmann et al. disclose a "minimal vector" containing the β-lactamase cassette from pBR322, an origin of replication and a multiple cloning site. This construct, however, still contains sequences into which a transposon can insert without intefering with the maintenance of the vector.

The object of the present invention was to develop new vectors for transposon-based sequencing in which undesired transposition events are minimized or not recovered, novel mwthods for DNA sequencing and methods for constructing the new vectors. Another object of the invention was a method for preparing novel cloning vectors.

One of the above objects is solved by a new vector which allows for negative selection of undesired transposition events which occur outside the nucleic acid region to be sequenced that has been introduced into the vector of the invention.

Such a vector for selective transposon insertion according to the invention (also referred to herein as "transposon-resistant vector") requires as essential elements only an origin of replication and a selectable marker gene, wherein the selectable marker gene does not need its own promoter but is operatively linked to a promoter present in the origin of replication of the vector. Such a vector should be substantially free of sequences which tolerate transposon insertion.

Preferably, the promoter is a divergent promoter. A "divergent promoter", as used herein, is a set of sequences present in an origin of replication which allows transcription into opposite directions. Preferably, these two sequences overlap. A preferred example of a promoter according to the invention is the RNA I promoter which is present in the origin of replication of many plasmids.

Replication via the ColE1 origin starts with the transcription of an RNA that initiates about 500bp upstream of the origin. The enzyme RNase H cleaves the transript at the origin generating a "primer" at which DNA synthesis is initiated. Primer initiation is regulated by an RNA I molecule which is initiated by the RNA I promoter in the primer region and which is complementary to the 5'-terminal region of the primer RNA. Thus, the basepairing between the primer RNA and the RNA I controls the availability of primer to initiate a cycle of replication.

It was surprising to find that contrary to previous reports, it was possible to delete the entire promoter of the selectable marker gene and to express the gene as a read-through RNA from the promoter for RNA I off the origin of replication. The inventors found that the RNA I terminator in most origins of replication is leaky and that consequently it is possible to obtain read-through transcription products into a gene placed adjacent the origin of replication. If this gene is a selectable marker gene, such as an antibiotic resistance gene, the corresponding protein is produced in sufficient amounts to allow selection of the transposon-resistant plasmid.

This fact permits the construction of an entirely new class of transposon-resistant vectors which are excellent tools for applications in which the disruption of vector sequences, e.g. by transposon insertion, is highly undesirable because any insertion into such a transposon-resistant vector will either occur within the selectable marker gene in which case the vector will no longer protect against the selection means, or it will occur within the origin of replication which will prevent propagation of the vector. In either case such transposon insertions will be selected against.

Preferably, the transposon-resistant vector according to the invention is based on a plasmid vector. In principle, any bacterial or bacteriophage origin of replication can be used. Examples of bacterial origins of replication comprise ColE1 (pMB1), p15A, RSF1030, CloDF13 and their derivatives. Preferred origins of replication are those found in ColE1 and pUC plasmids.

The origin of replication may be full-length but it is also possible to use truncated versions, such as the one shown in SEQ ID NO:3 which is derived from the origin of a pUC18 vector (ColE1). As long as the RNA I promoter is functional and capable of producing readthrough products and the maintenance of the plasmid is ensured, any derivative of such an origin can be used for the purposes of this invention.

The selectable marker gene preferably is an antibiotic resistance gene such as the TEM-1 β-lactamase gene (conferring resistance against ampicillin), chloramphenicol resistance gene, tetracycline resistance gene, kanamycin resistance gene, neomycin resistance gene, and other resistance markers which will be known to the person skilled in the art. As long as transcription of the resistance gene is not interfered with (e.g. by disrupting the reading frame) and as long as transposon insertion can still be selected for, there may be additional sequences present in the vector, e.g between the RNA I promoter and the antibiotic resistance gene.

The basis of the transposon-resistant vectors of the invention can be any known cloning vector containing an antibiotic resistance gene and one of the origins mentioned above. Preferably the vectors according to the invention are derived from pUC and/or pBLUESCRIPT vectors. General cloning vectors are described in Sambrook et al. "Molecular Cloning: A Laboratory Manual", Chapter 1, CSH Laboratory Press, 1989 and are commercially available from companies like Pharmacia or Stratagene.

The construction of such a vector according to the invention may be via conventional cloning methods or by other *in vitro* methods such as PCR. By designing appropriate primers, it is possible to amplify selectively the desired origin and marker gene sequences, and to link the two together to form a functional vector unit.

Preferably, the vector additionally contains at least one cloning site or polylinker. Single or multiple cloning sites can be engineered into the vector again by cloning or by other methods such as PCR. Linker sequences for introducing cloning sites are commercially available.

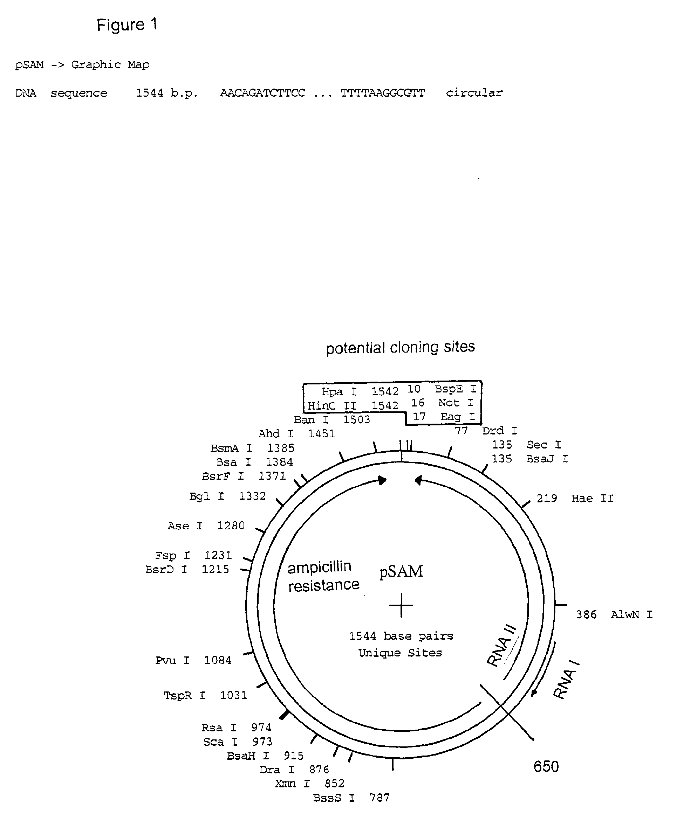

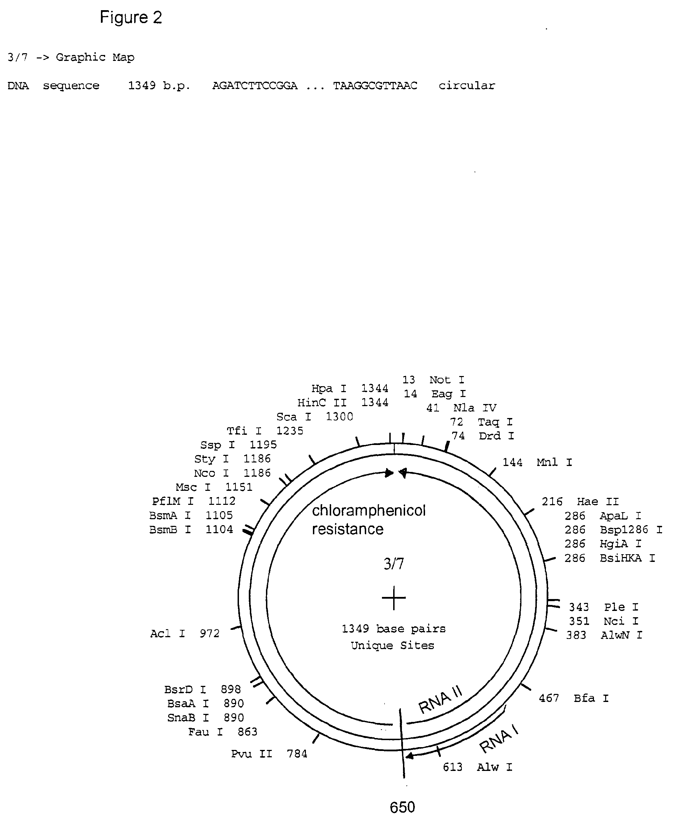

Examples of transposon-resistant vectors of the invention are the vectors pSAM and p3/7 depicted in Figures 1 and 2 whose sequences are shown in SEQ ID NOs:1 and 2. The construction of these vectors is described in detail in Example 1.

The invention also relates to a novel method for sequencing nucleic acids comprising the following steps:
(a) inserting a nucleic acid to be sequenced into a vector according to the invention, wherein a recombinant vector is obtained which contains a nucleic acid insert which tolerates transposon insertion,
(b) inserting a transposon into said recombinant vector wherein said transposon contains at least two unique sequences suitable for primer binding,
(c) selecting for a recombinant vector having a transposon insertion, and
(d) sequencing the nucleic acid insert using at least two primers capable of binding to said unique sequences.

The vector used for this sequencing method is a vector according to the invention, preferably pSAM or p3/7.

In a preferred embodiment, this method is carried out *in vivo* using host cells for the steps involving transposition events, i.e. the insertion (b) and selection (c) steps. An additional host cell may be used for step (a). Preferably, the host cells are of bacterial origin, such as *E.coli.*

The vector can be used in circular and linear form, depending on the system which is used for transposition. In general, for the use of bacterial host cells, circular vectors are required.

For the purposes of the above method the transposon in principle can be any transposon. Preferably, it has random or mainly random insertion sites. Preferred transposons are replicatively transposing elements, especially class II transposons, such as those of the Tn3 family, in particular *y*δ and derivatives thereof.

The transposon may be modified in various ways. It is advantageous to delete the enzymes required for transposition and additionally to delete non-essential sequences in order to reduce the size of the transposable element. The transposon used for the purposes of this invention preferably is *y*δ, and in particular the derivative *IS*102 which is known in the state of the art.

The transposon suitable for the method of the invention preferably further comprises a selectable marker gene. Examples of suitable drug resistance genes have already been mentioned above. In particular, it is useful to use a resistance gene which is copy number dependent, i.e. which confers resistance to varying amounts of the corresponding drug depending on how many copies of the gene are present in an individual cell. Thus, before transposition has taken place, the donor cell will contain only one copy of the marker. If a high copy number plasmid is used for transposon insertion the co-integrate molecule will replicate to high copy numbers and allow selection with higher amounts of the drug. For this purpose the marker should be different from the ampicillin resistance gene because it is not very copy number dependent and not very stringent. Preferred markers are chloramphenicol, kanamycin and streptomycin resistance genes.

Further, the transposon suitable for the sequencing methods of the invention has at least two unique sequences suitable for primer binding so that the transposon insertions can be employed for the priming of sequencing reactions. These sequences can be known primer binding sites.

When replicatively transposing elements are used, it is preferable to carry out the transposon insertion and selection steps in different host cells, wherein the first host cell is a donor cell capable of conjugative transfer of a co-integrate molecule to the second or recipient host cell.

The donor and recipient strains can be modified in various ways. It is often useful to provide the normally transposon-encoded transposase and resolvase enzymes *in trans* so that they are not encoded by the transposon but are present in the chromosome of the host cells. In general, the transposase will be provided by the donor and the resolvase by the recipient strain.

The recipient host cell is suitably a recA⁻ cell and carries a selectable marker gene. Optionally, the recipient may also lack endonuclease I, such as strain TOP10 (available from Invitrogen).

It is also possible to use more than one transposon, i.e. to use different transposons in order to avoid the problem of "cold spots", which are sequences into which the element will not transpose, which some sequences may have. Another possibility is to use several transposons which differ only by the unique primer binding sites. In each case, two or more different donor strains may be used containing different types of transposons. The donor strains can even be mixed together so that all the steps of the above method can be carried out in the same container.

The methods of the invention are carried out most efficiently when long stretches of DNA (long reads) can be sequenced, e.g. more than 1000 bases in one direction. Sequencing equipment particularly suited to this task is ARAKIS or Licor(TM) sequencing equipment.

A further aspect of the invention is a reagent kit, comprising a transposon-resistant vector according to the invention and a suitable transposon, preferably /S102. The reagent kit may additionally comprise host cells capable of being transformed with an F factor carrying the transposon, or host cells containing such an F factor, and optionally suitable media, buffers and/or selection compounds for culturing and/or selecting said host cells.

The vectors according to the invention are suitable as cloning vectors, e.g. in order to insert a DNA to be sequenced by the DNA sequencing method of the invention into a linearised vector molecule. Linearisation and ligation reactions are well known in the state of the art. In general, the linearised vector is dephosphorylated before it is mixed with the insert for ligation in order to prevent false positive results due to self-religation of the vector without insert. Unfortunately, such a dephosphorylation step is never 100 % efficient so that with difficult ligation reactions the number of religated vector molecules may still be too high. The present invention provides a method with which undesired religation events can be prevented with much higher efficiency than with conventional methods.

The invention provides a new method of generating vectors incapable of self-ligation because they lack 5'-phosphates. Any vector can be used for this method which comprises the following steps:
(a) providing a linearised vector,
(b) treating said vector with an exonuclease which degrades one strand of a double-stranded nucleic acid molecule in the 5' to 3' direction, wherein a mixture of overlapping single-stranded vector fragments is obtained,
(c) carrying out an extension reaction using the overlapping single-stranded fragments of (b) as templates and non-phosphorylated oligonucleotides as primers which bind to the 3' termini of the single-stranded vector fragments, obtaining double-stranded vector fragments, and
(d) melting the double-stranded vector fragments to form single-stranded overlapping vector fragments and corresponding complementary fragments,
(e) allowing the complementary overlapping single-stranded vector fragments to anneal and prime a further extension reaction to form double-stranded vector molecules lacking 5' phosphates.

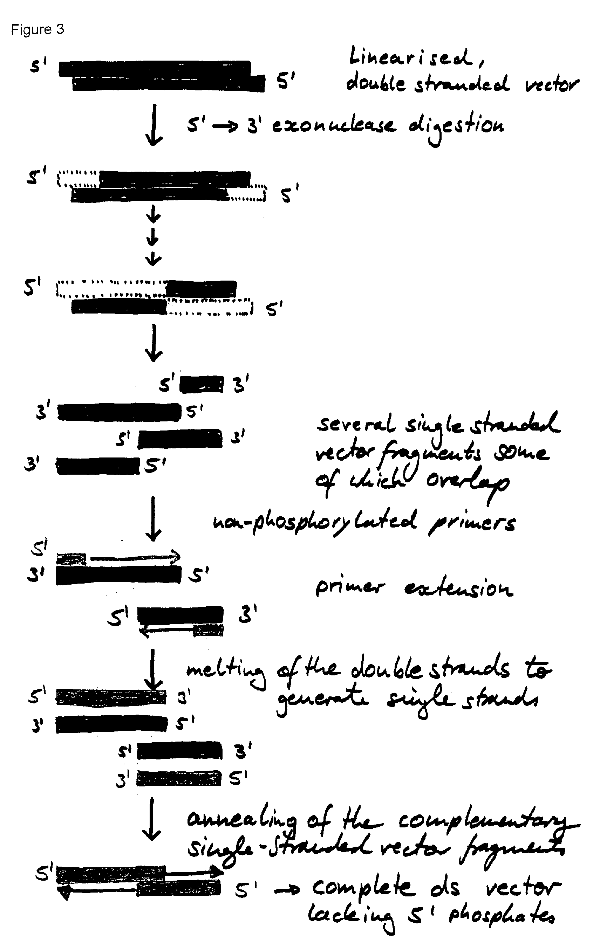

Preferably, the vectors of the invention are produced and/or propagated using this method. The starting vector should first be linearised (generating either blunt or sticky ends). This can be done with any suitable restriction enzyme. Then, an exonuclease such as λ exonuclease is used to generate single-stranded templates. This enzyme digests each strand of a double-stranded vector molecule from the 5'-termini. When the two enzymes meet (each on its own strand) the DNA molecule falls apart because there is no more double-stranded DNA left. The exonuclease will stop digesting because it only degrades one strand of a double-stranded molecule but not single strands. The two exonucleases may meet exactly in the middle of the linearised vector molecule but also at various other points depending on whether on λ exonuclease molecule started degrading on one strand before the second exonuclease had bound to the complementary strand. Thus, a population of identical linearised vector molecules will lead to a heterogeneous population of single-stranded fragments (vector "halves") some of which will overlap in the middle. These can be extended using short non-phosphorylated oligonucleotide primers which bind to the 3'-termini of the original linearised single stranded vector templates. The extension in turn leads to double-stranded vector fragments. These are then melted to generate single strands. The single-stranded fragments complementary to the original single-stranded vector fragments can then anneal to each other and subsequent extension steps and optional amplification steps with the same short primers will yield complete vector sequences. This method is schematically depicted in Figure 3.

The products will all lack 5'-phosphates. Only the few original exonuclease-digested vector sequences will have 5'-phosphates. Optionally, these can be destroyed by using a suitable restriction enzyme which can distiguish between the original vector DNA (e.g. because it is of bacterial origin) and the DNA produced by PCR amplification. One example of a suitable enzyme is Dpnl which cuts at the sequence GATC where the A is methylated at the 6 position (in *E. coli* all adenines in this sequences are methylated by *dam* methylase). So the original sequences are degraded and the remaining sequences are sequences with no 5'-phosphates.

The obvious advantage of cloning in this way is that all the colonies from a ligation reaction a vector prepared as described above and any insert will give colonies all of which will have inserts. Another advantage of this method is that all the steps can be carried out in the same test tube and using the same buffer.

### Description of Figures and Sequence Listing

- Figure 1: is a schematic representation of the transposonresistant vector pSAM according to the invention.
- Figure 2: is a schematic representation of the transposonresistant vector p3/7 according to the invention.
- Figure 3: is a schematic representation of the method for generating cloning vectors of the present invention which lack 5' phosphates.

- SEQ ID NO: 1: shows the transposon-resistant vector pSAM according to the invention.
- SEQ ID NO:2: shows the transposon-resistant vector p3/7 according of the invention.
- SEQ ID NO:3: shows the sequence of a truncated origin of replication (ColE1) used to drive transcription of the antibiotic resistance gene in pSAM and p3/7.
- SEQ ID NO:4: shows primer fh201.
- SEQ ID NO:5: shows primer fh202.
- SEQ ID NO:6: shows primer fh203.
- SEQ ID NO:7: shows primer fh204.
- SEQ ID NO:8: shows primer fh205.
- SEQ ID NO:9: shows primer fh206.
- SEQ ID NO:10: shows primer fh207.
- SEQ ID NO:11: shows primer gd1.
- SEQ ID NO:12: shows primer gd2.
- SEQ ID NO:13: shows primer fh37.
- SEQ ID NO:14: shows primer fh73.

The invention shall be further illustrated by the following non-limiting examples.

### Examples

### Example 1

### Construction of transposon-resistant vectors pSAM and p3/7 of the invention

Four transposon-resistant plasmid vectors were constructed, two with ampicillin resistance but differently sized origins and two with chloramphenicol resistance with the same origins of replication.

The following oligonucleotides were used:

SEQ ID NO:4 (fh201):
5'-GGAAGATCTCTTCCGCTTCCTCGCTCA-3'
SEQ ID NO:5 (fh202):
5'-GGAAGATCTCGTAGAAAAGATCAAAGGATCTTC-3'
SEQ ID NO:6 (fh203):
5'-GGAAGATCTGTTAACGCCTTAAAAAAATTACGCCCCGCC-3'
SEQ ID NO:7 (fh204):
5'-GGAAGATCTTCAGGAGCTAAGGAAGCTAAAATGG-3'
SEQ ID NO:8 (fh205):
5'-GGAAGATCTGAAAAAGGAAGAGTATGAGTATTCAAC-3'
SEQ ID NO:9 (fh206):
5'-GGAAGATCTGTTAACGCCTTAAAAAAATTACCAATGCTTAATCAGTGAGGCACC-
3'
SEQ ID NO:10 (fh207):
5'-GGAGCGGCCGCGTTGCTGGCGTTTTTCC-3'

The templates used were pUC18 and pBC KS+ (Stratagene). All primers except fh207 generate BgIII fragments (underlined sequence denotes BgIII site).

Primers fh201 and fh202 were used to PCR amplify the pUC origin of replication and primers fh203 and fh204 were used to amplify the chloramphenicol resistance gene open reading frame (ORF). The PCR products were digested with BgIII (recognition site AGATCT) and ligated together. All resulting clones had the fragments in the same orientation, with the RNA I promoter transcribing the resistance gene. A subsequent amplification with primer fh207 reduced the size of the pUC origin, and primers fh205 and fh206 were used to amplify, again as BgIII fragments, the β-lactamase ORF (encoding ampicillin resistance).

The minimal vectors can be prepared for use in cloning by standard methods:
These would include
1) digestion with a restriction enzyme (e.g. Hpa I, whose recognition site is GTTAAC) followed by
2) dephosphorylation with alkaline phosphatase and (optionally)
3) gel-purification of the digested, dephosphorylated vector DNA.

Alternatively, they can be prepared as described in Example 3 for efficient elimination of vector containing 5'-phosphates.

### Example 2

### DNA-Sequencing using a modified yδ transposon

Transposition of *y*δ into pSAM recombinants (for p3/7 recombinants, the same protocol is used substituting chloramphenicol at 15 micrograms per ml for ampicillin).
Day 1:
   1) Transform the recombinant pSAM clone into XL-1 B cells. These are available from Stratagene. Plate the transformation on ampicillin plates.
   2) TOP10 cells (available from Invitrogen) are needed on as the Streptomycin resistant recipient cells on day 2. Streak a plate in preparation (LB agar).
Day 2:
   1) Pick one colony of TOP10 into LB medium and grow overnight. These will be the recipient cells.
   2) Pick one XL-1B/pUC18 recombinant colony for each pUC18 clone used and grow in LB/ampicillin overnight.
Day 3:
   1) Perform conjugational transposition by mixing 100 microlitres of the XL-1 B recombinant culture with 100 microlitres of the TOP10 culture in 2mls of LB medium (without antibiotics) in a 15ml Falcon tube and mix (250-300 rpm) for 2 hours at 37°C.
   2) After the incubation, prepare 1:10 and 1:100 dilutions of the conjugation mix (in LB medium).
   3) Plate 100 microlitre and 200 microlitre aliquots of each dilution on ampicillin (100 micrograms per ml) and streptomycin (500 micrograms per ml) plates and incubate overnight at 37°C.
Day 4:
   Well isolated colonies should be visible on plates from at least one of the dilutions. Pick as many single colonies as required and grow overnight in LB medium with ampicillin (steptomycin is no longer necessary).

Sequence with gd1 and gd2 primers
(SEQ ID NO:11 (gd1): 5'-CAACGAATTATCTCCTT-3';
SEQ ID NO:12 (gd2): 5'-TCAATAAGTTATACCA-3'), using any standard protocol. Note that for cycle sequencing reactions, the annealing temperature for this pair of primers is 45°C. Ideally, both primers are used together; in this case, FITC-labelled gd1 and Cy5-labelled gd2 primers are used with unlabelled terminators.

### Example 3

### Preparing vectors lacking 5'-phosphate groups

A novel method of preparing the vector, such that non-recombinant clones are minimised, comprises the following steps:
1) linearising the vector DNA, using, for example, a restriction enzyme,
2) digestion of the vector using a 5' to 3' exonuclease, such as lambda exonuclease,
3) reassembly of the vector by PCR, using non-phosphorylated oligonucleotides,
4) blunt-ending of the vector PCR product (this step is not essential, but if Taq polymerase is used, it improves the cloning efficiency)
5) destruction of any remaining vector DNA (not generated by PCR) with the restriction enzyme Dpnl.

This was carried out as follows, using p3/7 as an example:
1) The vector was digested with a restriction enzyme Hpal; (this generates blunt-ends, cutting in the middle of the sequence GTTAAC, but it is not essential that the enzyme generates a blunt end).
   5 micrograms of p3/7 was digested in a volume of 40 microlitres with an eight-fold excess of Hpal enzyme (supplied by New England Biolabs) for one hour at 37°C in the manufacturer's Buffer 4 (see catalogue for details).
2) The linearised vector was digested by lambda exonuclease. This is a 5' to 3' exonuclease which degrades the vector to single-stranded fragments.
   5 microlitres, comprising 50 units of lambda exonuclease, was added to 25 microlitres of the Hpal digestion reaction and incubated for one hour at 37°C.
3) The fragmented vector was used as a template for PCR with the non-phosphorylated oligonucleotides
   fh37 (sequence: 5' GCGGCCGCGTTGCTGGCG 3') and fh73 (sequence: 5' AACTTATTACGCCCCGCCCTG 3').
   1 microlitre of the exonuclease reaction was used as the template, each non-phosphorylated oligonucleotide was used at 1 picomole per microlitre in a 50 microlitre PCR reaction using Pfu polymerase (supplied by Promega) under conditions recommended by the manufacturer. The annealing temperature was 55°C.
4) The resulting PCR product was blunt-ended (Pfu from *Pyrococcus furiosus* is a thermostable "proofreading" enzyme) and purified on a PCR purification column (supplied by Qiagen) used according to the manufacturer's instructions.
5) Any remaining parental vector was digested with Dpnl (25 units), in a final volume of 100 microlitres for three hours, according to the enzyme supplier's recommendations (New England Biolabs). The vector preparation was then gel-purified and was blunt-ended, devoid of 5'phosphate groups and ready for use as a cloning vector.
6) Note that
   a) the vector can be propagated entirely in vitro from this point by PCR and
   b) generation of non-recombinant clones (by illegitimate recombination recircularising the vector) is prevented, because such events would lead to deletion of essential vector elements.
7) The vector preparation was used to clone fragments of three BACs (bacterial artificial chromosomes) containing *Arabidopsis thaliana* DNA. The fragments were generated by partial digestion with Sspl. All randomly picked clones (of 96) had inserts of varying sizes. A number of these were sequenced using a modified *y*δ transposon containing "Universal" and "Reverse" primer binding sites engineered just inside the transposon ends.

## Claims

1. A vector for selective transposon insertion, comprising an origin of replication and a selectable marker gene, wherein the selectable marker gene is operatively linked to a promoter present in the origin of replication, and wherein said vector is substantially free of sequences which tolerate transposon insertion.

2. The vector according to claim 1, wherein the promoter is an RNA I promoter.

3. The vector according to claim 1 or 2, additionally containing at least one cloning site.

4. The vector according to any one of claims 1 to 3, wherein the origin of replication is derived from a ColE1, pMB1, p15A, pRSF1030 or pCloDF13 origin.

5. The vector according to claim 4, wherein the origin of replication is truncated.

6. The vector according to any one of claims 1 to 5, wherein the selectable marker gene is selected from the group consisting of genes coding for ampicillin resistance, chloramphenicol resistance, streptomycin resistance, tetracycline resistance, neomycin resistance and kanamycin resistance.

7. The vector according to any one of the previous claims, which is pSAM (SEQ ID NO:1) or p3/7 (SEQ ID NO:2).

8. A method for sequencing nucleic acids, comprising
(a) inserting a nucleic acid to be sequenced into the vector according to any one of claims 1 to 6, wherein a recombinant vector is obtained which contains a nucleic acid insert which tolerates transposon insertion,
(b) inserting a transposon into said recombinant vector wherein said transposon contains at least two unique sequences suitable for primer binding,
(c) selecting for a recombinant vector having a transposon insertion, and
(d) sequencing the nucleic acid insert using at least two primers capable of binding to said unique sequences.

9. The method according to claim 8, wherein said recombinant vector of step (a) is used in circular form.

10. The method according to claim 8 or 9, wherein said inserting (b) and selecting (c) steps and are carried out in a host cell.

11. The method according to claim 10, wherein said inserting step (b) is carried out in a donor host cell and said selecting step (c) is carried out in a recipient host cell.

12. The method according to claim 11, wherein the recombinant vector is transferred from the donor host cell to the recipient host cell by conjugation.

13. The method according to any one of claims 10 to 12, wherein the transposase and/or resolvase enzymes of the transposon are not encoded by the transposon but are provided by the host cell.

14. The method according to claim 13, wherein the transposase is provided by the donor host cell and the resolvase is provided by the recipient host cell.

15. The method according to claim 13 or 14, wherein the transposase is operatively linked to an inducible promoter.

16. The method according to claim 15, wherein said inducible promoter is the araB promoter.

17. The method according to any one of claims 10 to 16, wherein the second host cell lacks endonuclease I, is recA⁻ and carries a selectable resistance gene.

18. The method according to any one of claims 8 to 17, wherein the transposon is a class II transposon or a derivative thereof.

19. The method according to claim 18, wherein the transposon is the transposon *y*δ or a derivative thereof.

20. The method according to claim 19, wherein the transposon is *IS*102.

21. The method according to one of claims 8 to 19, wherein the transposon contains a selectable marker gene.

22. The method according to claim 21, wherein the selectable marker gene is selected from ampicillin resistance, chloramphenicol resistance, streptomycin resistance, tetracycline resistance, neomycin resistance and kanamycin resistance.

23. The method according to claim 22, wherein the selectable marker confers resistance in a copy number dependent fashion.

24. The method according to any one of claims 10 to 23, wherein step (b) is carried out in parallel in plurality of different donor host cells.

25. The method according to claim 24, wherein said different donor host cells contain different transposons.

26. The method according to claim 24, wherein the donor host cells contain transposons containing different unique site for primer binding.

27. The method according to one of claims 8 to 26, wherein the vector pSAM (SEQ ID NO:1) or the vector p3/7 (SEQ ID NO:2) is used as the transposon-resistant vector.

28. The method for constructing a cloning vector which is incapable of self-ligation, comprising
(a) providing a linearised vector,
(b) treating said vector with an exonuclease which degrades one strand of a double-stranded nucleic acid molecule in the 5' to 3' direction, wherein a mixture of overlapping single-stranded vector fragments is obtained,
(c) carrying out an extension reaction using the overlapping single-stranded fragments of (b) as templates and non-phosphorylated oligonucleotides as primers which bind to the 3' termini of the single-stranded vector fragments, obtaining double-stranded vector fragments, and
(d) melting the double-stranded vector fragments to form single-stranded overlapping vector fragments and corresponding complementary fragments,
(e) allowing the complementary overlapping single-stranded vector fragments to anneal and prime a further extension reaction to form double stranded vector molecules lacking 5' phosphates.

29. The method according to claim 28, additionally comprising
(f) specifically degrading the starting vector of step (a) and the fragments thereof of step (b), wherein the starting vector of step (a) is of different origin than the extended complementary vector fragments of step (d).

30. The method according to claim 29, wherein the vector of step (a) is of bacterial origin, and wherein in step (f) an enzyme degrading nucleic acids of bacterial origin is used.

31. The method according to any one of claims 28 to 30, wherein in step (b) λ exonuclease is used as the exonuclease.

32. A reagent kit, comprising a vector according to any one of claims 1 to 7, a suitable transposon and, optionally, host cells, media, buffers and/or selection compounds for culturing and/or selecting said host cells.


SEQUENCE LISTING

<110> European Molecular Biology Laboratory (EMBL)

<120> Vectors for use in transposon-based DNA sequencing
      methods

<130> 21222PEP Transposon-based sequencing

<140>
<141>

<160> 14

<170> PatentIn Ver. 2.1

<210> 1
<211> 1544
<212> DNA
<213> Artificial Sequence

<220>
<223> Description of Artificial Sequence: plasmid

<400> 1
aacagatctt ccggagcggc cgcgttgctg gcgtttttcc ataggctccg cccccctgac 60
gagcatcaca aaaatcgacg ctcaagtcag aggtggcgaa acccgacagg actataaaga 120
taccaggcgt ttccccctgg aagctccctc gtgcgctctc ctgttccgac cctgccgctt 180
accggatacc tgtccgcctt tctcccttcg ggaagcgtgg cgctttctca tagctcacgc 240
tgtaggtatc tcagttcggt gtaggtcgtt cgctccaagc tgggctgtgt gcacgaaccc 300
cccgttcagc ccgaccgctg cgccttatcc ggtaactatc gtcttgagtc caacccggta 360
agacacgact tatcgccact ggcagcagcc actggtaaca ggattagcag agcgaggtat 420
gtaggcggtg ctacagagtt cttgaagtgg tggcctaact acggctacac tagaaggaca 480
gtatttggta tctgcgctct gctgaagcca gttaccttcg gaaaaagagt tggtagctct 540
tgatccggca aacaaaccac cgctggtagc ggtggttttt ttgtttgcaa gcagcagatt 600
acgcgcagaa aaaaaggatc tcaagaagat cctttgatct tttctacgag atctgaaaaa 660
ggaagagtat gagtattcaa catttccgtg tcgcccttat tccctttttt gcggcatttt 720
gccttcctgt ttttgctcac ccagaaacgc tggtgaaagt aaaagatgct gaagatcagt 780
tgggtgcacg agtgggttac atcgaactgg atctcaacag cggtaagatc cttgagagtt 840
ttcgccccga agaacgtttt ccaatgatga gcacttttaa agttctgcta tgtggcgcgg 900
tattatcccg tattgacgcc gggcaagagc aactcggtcg ccgcatacac tattctcaga 960
atgacttggt tgagtactca ccagtcacag aaaagcatct tacggatggc atgacagtaa 1020
gagaattatg cagtgctgcc ataaccatga gtgataacac tgcggccaac ttacttctga 1080
caacgatcgg aggaccgaag gagctaaccg cttttttgca caacatgggg gatcatgtaa 1140
ctcgccttga tcgttgggaa ccggagctga atgaagccat accaaacgac gagcgtgaca 1200
ccacgatgcc tgtagcaatg gcaacaacgt tgcgcaaact attaactggc gaactactta 1260
ctctagcttc ccggcaacaa ttaatagact ggatggaggc ggataaagtt gcaggaccac 1320
ttctgcgctc ggcccttccg gctggctggt ttattgctga taaatctgga gccggtgagc 1380

gtgggtctcg cggtatcatt gcagcactgg ggccagatgg taagccctcc cgtatcgtag 1440
ttatctacac gacggggagt caggcaacta tggatgaacg aaatagacag atcgctgaga 1500
taggtgcctc actgattaag cattggtaat ttttttaagg cgtt 1544

<210> 2
<211> 1349
<212> DNA
<213> Artificial Sequence

<220>
<223> Description of Artificial Sequence: plasmid

<400> 2
agatcttccg gagcggccgc gttgctggcg tttttccata ggctccgccc ccctgacgag 60
catcacaaaa atcgacgctc aagtcagagg tggcgaaacc cgacaggact ataaagatac 120
caggcgtttc cccctggaag ctccctcgtg cgctctcctg ttccgaccct gccgcttacc 180
ggatacctgt ccgcctttct cccttcggga agcgtggcgc tttctcatag ctcacgctgt 240
aggtatctca gttcggtgta ggtcgttcgc tccaagctgg gctgtgtgca cgaacccccc 300
gttcagcccg accgctgcgc cttatccggt aactatcgtc ttgagtccaa cccggtaaga 360
cacgacttat cgccactggc agcagccact ggtaacagga ttagcagagc gaggtatgta 420
ggcggtgcta cagagttctt gaagtggtgg cctaactacg gctacactag aaggacagta 480
tttggtatct gcgctctgct gaagccagtt accttcggaa aaagagttgg tagctcttga 540
tccggcaaac aaaccaccgc tggtagcggt ggtttttttg tttgcaagca gcagattacg 600
cgcagaaaaa aaggatctca agaagatcct ttgatctttt ctacgagatc ttcaggagct 660
aaggaagcta aaatggagaa aaaaatcact ggatatacca ccgttgatat atcccaatgg 720
catcgtaaag aacattttga ggcatttcag tcagttgctc aatgtaccta taaccagacc 780
gttcagctgg atattacggc ctttttaaag accgtaaaga aaaataagca caagttttat 840
ccggccttta ttcacattct tgcccgcctg atgaatgctc atccggaatt acgtatggca 900
atgaaagacg gtgagctggt gatatgggat agtgttcacc cttgttacac cgttttccat 960
gagcaaactg aaacgttttc atcgctctgg agtgaatacc acgacgattt ccggcagttt 1020
ctacacatat attcgcaaga tgtggcgtgt tacggtgaaa acctggccta tttccctaaa 1080
gggtttattg agaatatgtt tttcgtctca gccaatccct gggtgagttt caccagtttt 1140
gatttaaacg tggccaatat ggacaacttc ttcgcccccg ttttcaccatg ggcaaatatt 1200
atacgcaagg cgacaaggtg ctgatgccgc tggcgattca ggttcatcat gccgtttgtg 1260
atggcttcca tgtcggcaga atgcttaatg aattacaaca gtactgcgat gagtggcagg 1320
gcggggcgta atttttttaa ggcgttaac 1349

<210> 3
<211> 631
<212> DNA
<213> Escherichia coli

<400> 3
ggccgcgttg ctggcgtttt tccataggct ccgcccccct gacgagcatc acaaaaatcg 60
acgctcaagt cagaggtggc gaaacccgac aggactataa agataccagg cgtttccccc 120
tggaagctcc ctcgtgcgct ctcctgttcc gaccctgccg cttaccggat acctgtccgc 180

ctttctccct tcgggaagcg tggcgctttc tcatagctca cgctgtaggt atctcagttc 240
ggtgtaggtc gttcgctcca agctgggctg tgtgcacgaa ccccccgttc agcccgaccg 300
ctgcgcctta tccggtaact atcgtcttga gtccaacccg gtaagacacg acttatcgcc 360
actggcagca gccactggta acaggattag cagagcgagg tatgtaggcg gtgctacaga 420
gttcttgaag tggtggccta actacggcta cactagaagg acagtatttg gtatctgcgc 480
tctgctgaag ccagttacct tcggaaaaag agttggtagc tcttgatccg gcaaacaaac 540
caccgctggt agcggtggtt tttttgtttg caagcagcag attacgcgca gaaaaaaagg 600
atctcaagaa gatcctttga tcttttctac g 631

<210> 4
<211> 27
<212> DNA
<213> Artificial Sequence

<220>
<223> Description of Artificial Sequence: Primer

<400> 4
ggaagatctc ttccgcttcc tcgctca 27

<210> 5
<211> 33
<212> DNA
<213> Artificial Sequence

<220>
<223> Description of Artificial Sequence: Primer

<400> 5
ggaagatctc gtagaaaaga tcaaaggatc ttc 33

<210> 6
<211> 39
<212> DNA
<213> Artificial Sequence

<220>
<223> Description of Artificial Sequence: Primer

<400> 6
ggaagatctg ttaacgcctt aaaaaaatta cgccccgcc 39

<210> 7
<211> 34

<212> DNA
<213> Artificial Sequence

<220>
<223> Description of Artificial Sequence: Primer

<400> 7
ggaagatctt caggagctaa ggaagctaaa atgg                                34

<210> 8
<211> 36
<212> DNA
<213> Artificial Sequence

<220>
<223> Description of Artificial Sequence: Primer

<400> 8
ggaagatctg aaaaaggaag agtatgagta ttcaac                              36

<210> 9
<211> 54
<212> DNA
<213> Artificial Sequence

<220>
<223> Description of Artificial Sequence: Primer

<400> 9
ggaagatctg ttaacgcctt aaaaaaatta ccaatgctta atcagtgagg cacc          54

<210> 10
<211> 28
<212> DNA
<213> Artificial Sequence

<220>
<223> Description of Artificial Sequence: Primer

<400> 10
   ggagcggccg cgttgctggc gtttttcc                                      28

<210> 11
<211> 17

<212> DNA
<213> Artificial Sequence

<220>
<223> Description of Artificial Sequence: primer

<400> 11
caacgaatta tctcctt                                                    17



<210> 12
<211> 16
<212> DNA
<213> Artificial Sequence

<220>
<223> Description of Artificial Sequence: primer

<400> 12
tcaataagtt atacca                                                     16



<210> 13
<211> 18
<212> DNA
<213> Artificial Sequence

<220>
<223> Description of Artificial Sequence: primer

<400> 13
gcggccgcgt tgctggcg                                                   18



<210> 14
<211> 21
<212> DNA
<213> Artificial Sequence

<220>
<223> Description of Artificial Sequence: primer

<400> 14
aacttattac gccccgccct g                                               21